# FASCICULE DE BREVET EUROPEEN

(11) **EP 1 917 529 B1**
(45) Date de publication et mention de la délivrance du brevet: **30.06.2010**
(21) Numéro de dépôt: 06808820.2
(22) Date de dépôt: 23.08.2006
(51) Int. Cl.: G01N 33/558

(54) **DOSAGE D'UN ANALYTE PAR IMMUNOCHROMATOGRAPHIE AVEC MIGRATION LATERALE**
TESTEN VON ANALYTEN MITTELS IMMUNCHROMATOGRAPHIE MIT LATERALER MIGRATION
ANALYTE ASSAYING BY MEANS OF IMMUNOCHROMATOGRAPHY WITH LATERAL MIGRATION

(30) Priorité: 23.08.2005 FR 0508685
(43) Date de publication de la demande: 07.05.2008
(73) Titulaire: Vedalab, 61000 Alençon (FR)
(72) Inventeur: DONATI, Raphaël, F-61250 Radon (FR)
(74) Mandataire: Chevalier, Renaud Philippe
(86) Numéro de dépôt international: PCT/IB2006/002308
(87) Numéro de publication internationale: WO 2007/023372

(56) Documents cités:
- WO-A-2004/088320
- DE-A1- 10 054 093
- US-A- 5 962 339
- US-A1- 2005 112 779
- US-B1- 6 664 114

## Description

La présente invention concerne un dispositif immunochromatographique à migration latérale permettant simultanément la détermination d'un analyte dans un échantillon liquide par un test sandwich et par un test de compétition.

Des dispositifs immunochromatographiques à migration latérale sont par exemple décrits dans le brevet EP 0 284 232. Ces dispositifs mettent en oeuvre un moyen de diffusion capillaire sous la forme d'un support solide poreux au sein duquel l'échantillon et les réactifs migrent par diffusion capillaire. Ces dispositifs intègrent ou comprennent ainsi un support solide poreux (ou immunochromatographique) comportant une première zone portant sous forme lyophilisée, sèche, ou déshydratée, un réactif de liaison spécifique de l'analyte conjugué à un marqueur visible et/ou mesurable, et une zone de détection sur laquelle est immobilisé un réactif de capture spécifique de l'analyte. Le réactif de liaison spécifique de l'analyte est immobile sous forme sèche, mais devient mobile dans le support solide à l'état humide. Ainsi, lorsque le support solide est mis en contact avec un échantillon liquide, ce dernier migre par diffusion capillaire dans ce support entraînant le réactif de liaison spécifique de l'analyte conjugué au marqueur visible et/ou mesurable. L'échantillon et le réactif de liaison spécifique de l'analyte migrent par diffusion capillaire dans le support solide jusqu'à la zone de détection portant le réactif de capture spécifique de l'analyte immobilisé.

On connaît de WO 2004/088320 des procédés immunochromtographiques en phase solide dans lesquels le réactif de liaison, conjugué à un marqueur visible et/ou mesurable est ajouté extemporanément sous forme liquide.

Ces dispositifs permettent classiquement la détection de l'analyte, soit par un test sandwich, soit par un test de compétition.

Dans un test sandwich, le réactif de liaison marqué se lie à l'analyte, et ce dernier est immobilisé sur le support solide par le réactif de capture. La présence ou l'absence de l'analyte dans l'échantillon est mesurée par la présence ou non d'un signal visible ou mesurable,au niveau du réactif de capture.

Dans un test de compétition, l'analyte et le réactif de liaison sont en compétition pour se lier au réactif de capture. La présence ou l'absence de l'analyte dans l'échantillon est mesurée par l'absence ou non d'un signal visible ou mesurable, au niveau du réactif de capture.

Les brevets EP 0 291 194, EP 0 560 411 et EP 0 560 410 décrivent des dispositifs dans lesquels le support solide immunochromatographique en une seule partie est incorporé dans un boîtier pourvu d'une ouverture pour le dépôt de l'échantillon liquide, et d'une fenêtre d'observation pour la lecture du résultat.

Le brevet EP 1 091 808 décrit des dispositifs améliorés comprenant également un support solide poreux en plusieurs parties, avec un organe de captation de l'échantillon liquide, mobile, permet une meilleure collecte de l'échantillon.

Ces dispositifs peuvent être adaptés à un usage unique et domestique. En effet, ils sont d'un usage facile et rapide, ne nécessitant que très peu de manipulations puisque tous les réactifs sont intégrés ou compris dans le dispositif.

Cependant, ces tests immunochromatographiques en phase solide ne permettent pas une détermination quantitative de l'analyte dans l'échantillon.

En outre, en test sandwich, on observe pour certains analytes un effet "crochet", ou effet « Hook ». L'effet crochet est un effet indésirable bien connu dans les tests immunologiques. Il se produit lorsque l'analyte est présent dans l'échantillon à une concentration très élevée. L'effet crochet peut alors conduire à des faux négatifs, concluant de façon aberrante à l'absence de l'analyte dans l'échantillon.

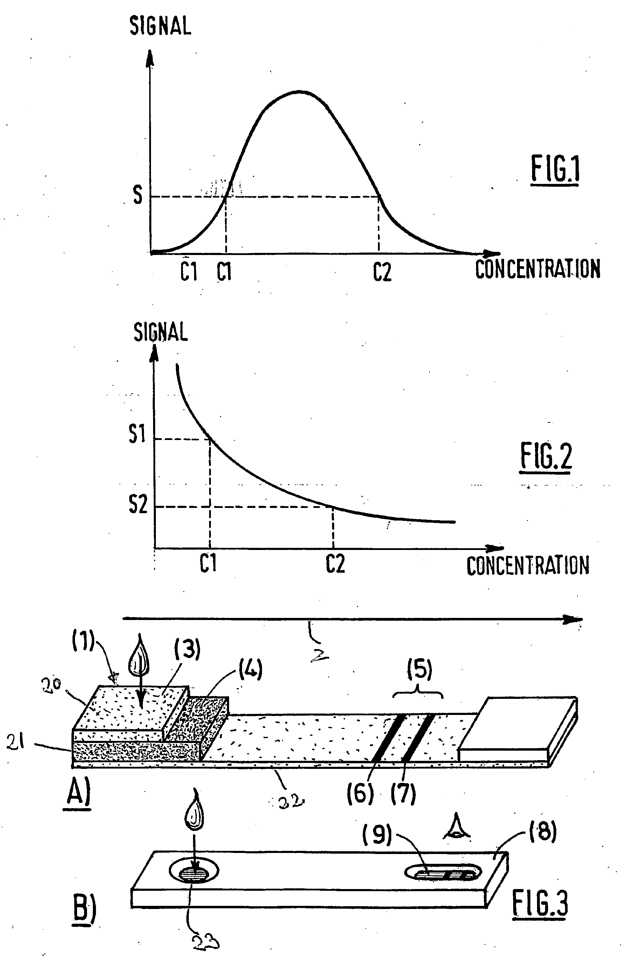

Les analytes présentant un effet crochet lors de dosages immunologiques de type sandwich présentent des courbes signal/concentration du type courbe de Gauss (voir figure 1). La figure 1 montre qu'à un signal donné (S) correspondent deux concentrations possibles de l'analyte (C1 et C2), l'une faible (C1) et l'autre élevée (C2) lors de la lecture du résultat à un temps défini.

Les courbes signal/concentration pour les dosages par compétition sont représentées à la figure 2. Pour un test par compétition, il y a bien l'obtention de deux signaux différents (S1 et S2) pour deux concentrations respectivement différentes (C1 et C2) de l'analyte à doser. Cependant, les tests par compétition montrent également très rapidement leurs limites, car il y a une extinction du signal à des concentrations relativement peu élevées d'analyte.

Une solution communément adoptée pour remédier à ces inconvénients des tests sandwich et des tests de compétition consiste à doser l'analyte, à partir d'une gamme de dilution de l'échantillon liquide. L'utilisation d'une gamme de dilution ne convient cependant pas un usage domestique. En outre, l'utilisation d'une gamme de dilution de l'échantillon nécessite des manipulations supplémentaires et une consommation accrue de dispositifs de test à usage unique, puisque chaque échantillon est testé plusieurs fois à différentes dilutions.

Conformément au document DE 10054093, on propose un dispositif d'immuno-filtration constitué de deux parties distinctes ou indépendantes, affectées respectivement à un test de type sandwich et à un test de type compétition. Avec ce dispositif, un réactif de liaison spécifique conjugué à un marqueur fluorescent est mélangé extemporanément avec l'analyte. Le mélange obtenu est ensuite divisé en deux portions. Une première portion est filtrée dans la partie sandwich, et réagit avec un deuxième réactif de liaison, immobilisé, et une deuxième portion est filtrée dans la partie compétition du dispositif, et réagit avec un réactif de capture, immobilisé, identique à l'analyte, ou un analogue de l'analyte. Les signaux fluorescents respectivement obtenus permettent la détermination de l'analyte.

Ce dispositif n'est pas susceptible d'améliorer la sensibilité ou précision de la détermination de l'analyte, car chaque test élémentaire travaille avec la concentration de départ du réactif de liaison spécifique.

Conformément au document US 2005/0112729 (cf. en particulier paragraphe 0063 et revendication 1), on a décrit un dispositif, pour la détermination d'un complexe entre un analyte et un premier réactif de liaison spécifique, marqué, ce dispositif comprenant :
- un moyen de diffusion capillaire,
- dans une zone dite de compétition du moyen de diffusion capillaire, un deuxième réactif de liaison spécifique de l'analyte, immobilisé, et complexé avec un ligand, également marqué,
- dans une zone dite de détection du moyen de diffusion capillaire; un réactif de capture, immobilisé, susceptible de se lier, aussi bien au complexe précité qu'au ligand marqué, ce dernier étant susceptible d'être déplacé ou décomplexé dans la zone de compétition par l'écoulement dudit complexe sur le moyen de diffusion capillaire.

La détermination de l'analyte est obtenue à partir des signaux obtenus dans les zones de compétition et détection.

Le format de ce test est complexe à mettre en oeuvre, et ne se justifie que pour des déterminations de l'analyte de grande précision.

Le document US-A-5 962 339 décrit un test comportant une zone 5 dite « threshold » et une zone 6 dite « détection ». Le dispositif décrit ne propose pas simultanément sur la même membrane un dosage de l'analyte par sandwich et un dosage de l'analyte par compétition.

La présente invention a pour objet de remédier aux inconvénients des dispositifs discutés précédemment. En particulier, la présente invention a pour objet, d'une part un dispositif de test, et d'autre part un mode d'emploi de ce dernier, permettant un détermination de l'analyte, avec un degré de fiabilité et/ou précision suffisant, en toutes circonstances, c'est-à-dire quelle que soit la concentration de départ de ce dernier, en particulier lorsque cette concentration est relativement forte ou relativement faible.

Selon l'invention, par référence à la figure 3, on propose un dispositif de détermination d'un analyte dans un échantillon liquide, comportant :
- un moyen de diffusion capillaire 1, par migration latérale déterminant une direction de référence 2 de diffusion capillaire, comportant une zone de dépôt 3 de l'échantillon liquide, et une zone de détection 5 de l'analyte, disposée en aval de ladite zone de dépôt,
- un premier réactif 4 de liaison spécifique de l'analyte, conjugué à un marqueur visible et/ou mesurable, libre de migrer à l'état humide, par diffusion capillaire, dans le moyen de diffusion capillaire 1, selon la direction de référence, la zone de disposition du premier réactif étant confondue avec la zone de dépôt (3) de l'échantillon, ou étant disposée en aval de cette dernière, mais en amont de la zone de détection (5),
- un deuxième réactif 6 de liaison spécifique de l'analyte, immobilisé, dans la zone de détection 5,
caractérisé en ce que la zone de détection 5 comporte un troisième réactif contitué par l'analyte, ou un analogue de l'analyte, immobilisé, et disposé à distance du deuxième réactif 6 de liaison spécifique en aval de celui-ci.

Ce dispositif présente, de manière préférentielle, l'une ou plusieurs des caractéristiques secondaires suivantes, considérées seules ou en combinaison, toujours expliquées par référence à la figure 3 :
- ce dispositif peut comporter un organe de captation de l'échantillon liquide, indépendant du moyen de diffusion capillaire, susceptible d'être amené en contact de la zone de dépôt, pour un écoulement dudit échantillon dans ladite zone de dépôt.
- le moyen de diffusion capillaire 1 peut comporter une zone de disposition du premier réactif (4) de liaison, à l'état sec et libre, dans ou sur ledit moyen de diffusion capillaire ;
- le deuxième réactif 6 de liaison spécifique, et/ou l'analyte 7, ou l'analogue de analyte, sont disposés par exemple sur le moyen de diffusion capillaire selon une ligne transversale à la direction de référence.
- le moyen de diffusion capillaire peut comporter une zone de contrôle (C), en aval de la zone de détection 5.

Selon la présente invention, on entend par "moyen de diffusion capillaire" tout moyen constituant ou agissant en tant qu'unité de diffusion capillaire continue, par migration latérale (c'est-à-dire perpendiculairement à l'épaisseur du ou des matériaux capillaires mis en oeuvre pour la diffusion capillaire). Un tel moyen de diffusion capillaire est par exemple un support alllongé selon la direction et/ou le sens de la diffusion capillaire (migration latérale), constitué par un seul et même matériau capillaire ou poreux, ou par plusieurs éléments ou matériaux capillaires différents, agencés les uns par rapport aux autres, par exemple par chevauchement, pour obtenir une continuité d'écoulement capillaire d'un élément ou d'un matériau à un autre, selon la direction de diffusion capillaire.

Un tel moyen de diffusion capillaire détermine une direction et/ou sens de diffusion capillaire de tout liquide qui est reçu ou déposé à une extrémité, vers l'autre extrémité dudit moyen.

La diffusion capillaire considérée selon la présente invention, ou immunochromatographique par migration latérale, est à distinguer de celle mise en oeuvre dans les techniques d'immuno-filtration, selon lesquelles les liquides perméent dans l'épaisseur du ou des matériaux de filtration, poreux.

Selon la présente invention, l'analyte, ou l'analogue de l'analyte considéré, constitue un troisième réactif, en sus des premier et deuxième réactifs de liaison. Ce troisième réactif est fixé ou immobilisé dans la zone de détection, par tous moyens appropriés. Ce troisième réactif sur le moyen de diffusion capillaire est disponible pour réagir spécifiquement avec le premier réactif de liaison spécifique, à l'exclusion de toute autre entité. En particulier, une fois déposé sur le moyen de diffusion capillaire, il n'est pas complexé avec toute autre entité, ou réactif, par exemple conjugué avec un marqueur visible et/ou mesurable.

Un tel dispositif peut être mis en oeuvre selon le procédé ou mode d'emploi défini ci-après, par référence à la figure 3 :
- on dépose dans la zone de dépôt du moyen de diffusion capillaire, l'échantillon liquide,
- on attend un temps suffisant pour la migration par diffusion capillaire de l'échantillon liquide jusqu' à la zone de détection 5,
- on observe dans la zone de détection la mesure dans laquelle, d'une part le réactif 4 de liaison spécifique complexé avec l'analyte se fixe au deuxième réactif 6 de liaison spécifique, en obtenant un premier signal, et d'autre part le premier réactif de liaison non complexé se fixe au troisième réactif constitué par l'analyte 7, ou analogue de analyte, en obtenant un deuxième signal,
- on détermine l'analyte à partir des premier et deuxième signaux.

Préférentiellement, selon l'invention, la quantité du premier réactif 4 de liaison est excédentaire par rapport à la quantité de l'analyte présente dans l'échantillon liquide, ou du nombre de sites de complexation avec te premier réactif de liaison, présents dans ledit échantillon liquide.

Dans un mode qualitatif, une concentration faible en analyte est détectée par l'absence du premier signal et la présence du second signal ; et une concentration importante en analyte est détectée par l'absence des deux signaux. Une concentration normale de l'analyte est détectée par la présence des premier et second signaux.

Avantageusement, les dispositifs et/ou les procédés selon la présente invention permettent de doser des analytes faiblement ou très fortement concentrés dans un échantillon sans obtenir de résultats faux positifs
ou faux négatifs. En outre, de façon surprenante, les dispositifs et/ou les procédés selon la présente invention permettent le dosage quantitatif de l'analyte.

Les dispositifs et/ou les procédés selon l'invention sont particulièrement adaptés au dosage d'analytes présentant un effet crochet important comme l'hormone de grossesse (hCG), la protéine C-réactive, l'albumine, etc.

### Description de l'invention

La présente invention concerne donc un dispositif de détermination d'un analyte dans un échantillon liquide, comportant un moyen de diffusion capillaire (1) comprenant dans la direction de diffusion capillaire (2) :
a) une zone de dépôt ou réception de l'échantillon (3) ;
b) un réactif de liaison spécifique de l'analyte conjugué à un marqueur visible et/ou mesurable, libre de migrer par diffusion capillaire à l'état humide dans le moyen de diffusion capillaire (1) ;
c) une zone de détection de l'analyte (5) ;
dans lequel la zone de détection de l'analyte (5) comprend, successivement, dans la direction de diffusion capillaire (2), d'une part un deuxième réactif de liaison spécifique de l'analyte (6) immobilisé, et d'autre part l'analyte, ou un analogue de l'analyte, immobilisé (7) ; de telle sorte que la premier réactif de liaison spécifique de l'analyte (4) et le deuxième réactif de liaison spécifique de l'analyte (6) permettent la détermination de l'analyte dans l'échantillon liquide par un test sandwich, alors que le premier réactif de liaison spécifique de l'analyte (4) et l'analyte, ou l'analogue de l'analyte, immobilisé (7) permettent la détermination de l'analyte dans l'échantillon liquide par un test de compétition.

Dans un mode de réalisation, le premier réactif de liaison spécifique de l'analyte (4), conjugué à un marqueur visible et/ou mesurable, est déposé à l'état sec sur le support, et l'invention concerne donc un dispositif de détermination d'un analyte dans un échantillon liquide comportant un moyen de diffusion capillaire (1) comprenant, dans la direction de diffusion capillaire (2) :
a) une zone de dépôt de l'échantillon (3) ;
b) un premier réactif de liaison spécifique de l'analyte (4), conjugué à un marqueur visible et/ou mesurable, déposé à l'état sec et libre de migrer par diffusion capillaire à l'état humide dans le moyen de diffusion capillaire (1) ;
c) une zone de détection de l'analyte (5) ;
dans lequel la zone de détection de l'analyte (5) comprend, successivement, dans la direction de diffusion capillaire (2), un deuxième réactif de liaison spécifique de l'analyte (6) immobilisé, et l'analyte, ou un analogue de l'analyte, immobilisé (7) ; de telle sorte que le premier réactif de liaison spécifique de l'analyte (4) et le deuxième réactif de liaison spécifique de l'analyte (6) permettent la détermination de l'analyte dans l'échantillon liquide par un test sandwich, alors que le premier réactif de liaison spécifique de l'analyte (4) et l'analyte, ou l'analogue de l'analyte, immobilisé (7) permettent la détermination de l'analyte dans l'échantillon liquide par un test de compétition.

Dans un autre mode de réalisation, le premier réactif de liaison spécifique de l'analyte (4), conjugué à un marqueur visible et/ou mesurable, est ajouté extemporanément sous forme de réactif sous forme liquide.

Par « réactif sous forme liquide», on entend tout réactif dans lequel le réactif de liaison est en solution ou en suspension. La préparation du réactif de liaison conjugué au marqueur visible et/ou mesurable sous forme liquide se fait selon des techniques décrites dans la littérature. Habituellement, le réactif de liaison conjugué est en solution ou en suspension dans une solution saline tamponnée. Cette solution peut également comprendre des agents stabilisants et d'autres composés tels que des anti-bactériens ou des anti-fongiques. Parmi les agents stabilisants on citera par exemple la sérum albumine bovine (BSA) et la caséine.

Dans certains procédés selon la présente invention, un diluant est de plus utilisé lorsque l'échantillon liquide est du plasma, du sérum ou du sang total par exemple. Ce diluant migre dans le support solide entraînant l'échantillon, et le réactif de liaison marqué. Typiquement, ce diluant est composé d'une solution saline tamponnée, il peut également comprendre un détergent ou tout autre composant nécessaire à la réaction.

De préférence, le premier réactif de liaison spécifique de l'analyte (4) est conjugué à un marqueur particulaire.

Préférentiellement, le moyen de diffusion capillaire (1) est une bandelette chromatographique fixée sur un support rigide.

Dans un mode de réalisation préféré, le moyen de diffusion capillaire (1) est intégré dans un support de préhension (8) pourvu d'au moins une fenêtre d'observation (9) permettant d'observer la zone de détection (5).

Par « réactif de liaison », on entend toute entité chimique, biochimique, ou biologique, susceptible de se lier spécifiquement avec l'analyte, ou avec le réactif de capture en compétition avec l'analyte.

Par « lier » ou « liaison », on entend toute liaison forte, par exemple covalente, ou toute liaison faible, par exemple du type antigène/anticorps ou avidine/streptavidine.

Le réactif de liaison est par exemple un anticorps, un antigène ou un acide nucléique.

Dans un test par compétition, le réactif de liaison est en phase solide et fixé à l'analyte lui-même ou un analogue approprié de l'analyte. Par analogue approprié de l'analyte, on entend toute entité se liant de manière spécifique au premier réactif de liaison spécifique de l'analyte, en compétition avec l'analyte.

Dans un test de type sandwich, le premier réactif de liaison marqué est un anti-analyte conjugué à un marqueur visible et/ou mesurable, par exemple un anticorps spécifique de l'analyte, conjugué audit marqueur.

Dans un test de type sandwich, le deuxième réactif de liaison se lie de façon spécifique à l'analyte. Le deuxième réactif de liaison marqué est donc également un anti-analyte, par exemple un anticorps spécifique de l'analyte conjugué à un marqueur visible et/ou mesurable.

Par « marqueur visible et/ou mesurable », on entend tout procédé de marquage permettant une détection directe ou indirecte à l'oeil nu, ou à l'aide d'un appareil, en raison de l'émission d'un signal, ledit signal étant par exemple, une fluorescence, une coloration, une présence d'isotope, ou un signal magnétique. On citera par exemple les marqueurs particulaires colorés comme l'or colloïdal, ou fluorescents, les particules de latex colorées, les particules de latex fluorescentes et les particules conjuguées à l'avidine et à la streptavidine.

La présente invention concerne donc un dispositif de détermination d'un analyte dans un échantillon liquide, comportant un moyen de diffusion capillaire (1), comprenant dans la direction de diffusion capillaire (2) :
a) une zone de dépôt ou de réception de l'échantillon (3) ;
b) un premier anticorps spécifique de l'analyte (4), conjugué à un marqueur visible et/ou mesurable, libre de migrer par diffusion capillaire à l'état humide dans le moyen de diffusion capillaire (1) ;
c) une zone de détection de l'analyte (5) ; dispositif dans lequel la zone de détection de l'analyte (5) comprend, successivement, dans la direction de diffusion capillaire (2), d'une part un deuxième anticorps spécifique de l'analyte (6) immobilisé, et d'autre part l'analyte, ou un analogue de l'analyte, immobilisé (7) ; de telle sorte que le premier anticorps spécifique de l'analyte (4) et le deuxième anticorps spécifique de l'analyte (6) permettent la détermination de l'analyte dans l'échantillon liquide par un test sandwich, alors que, d'une part le premier anticorps spécifique de l'analyte (4), et d'autre part l'analyte, ou l'analogue de l'analyte, immobilisé (7) permettent la détermination de l'analyte dans l'échantillon liquide par un test de compétition.

Dans un mode de réalisation, le premier anticorps spécifique de l'analyte (4), conjugué à un marqueur visible et/ou mesurable est déposé à l'état sec sur le support, et l'invention concerne alors un dispositif de détermination d'un analyte dans un échantillon liquide, comportant un moyen de diffusion capillaire (1), et comprenant dans la direction de diffusion capillaire (2) :
a) une zone de dépôt ou réception de l'échantillon (3) ;
b) un premier anticorps spécifique de l'analyte (4), conjugué à un marqueur visible et/ou mesurable, déposé à l'état sec et libre de migrer par diffusion capillaire à l'état humide dans le moyen de diffusion capillaire (1) ;
c) une zone de détection de l'analyte (5) ;
   dispositif dans lequel la zone de détection de l'analyte (5) comprend, successivement, dans la direction de diffusion capillaire (2), d'une part un deuxième anticorps spécifique de l'analyte (6) immobilisé, et d'autre part l'analyte, ou un analogue de l'analyte, immobilisé (7) ; de telle sorte que le premier anticorps spécifique de l'analyte (4) et le deuxième anticorps spécifique de l'analyte (6) permettent la détermination de l'analyte dans l'échantillon liquide par un test sandwich, alors que, d'une part le premier anticorps spécifique de l'analyte (4), et d'autre part l'analyte, ou l'analogue de l'analyte, immobilisé (7), permettent la détermination de l'analyte dans l'échantillon liquide par un test de compétition.

Dans un autre mode de réalisation, le premier anticorps spécifique de l'analyte (4), conjugué à un marqueur visible et/ou mesurable, est ajouté extemporanément sous forme de réactif sous forme liquide.

De préférence, le premier anticorps spécifique de l'analyte (4) est conjugué à un marqueur visible et/ou mesurable.

Préférentiellement, le moyen de diffusion capillaire (1) est une bandelette chromatographique fixée sur un support rigide.

Dans un mode de réalisation préféré, le moyen de diffusion capillaire (1) est intégré dans un support de préhension (8) pourvu d'au moins une fenêtre d'observation (9) permettant d'observer la zone de détection (5).

L'invention concerne aussi un procédé de détermination d'un analyte dans un échantillon liquide, mettant en oeuvre un dispositif tel que défini précédemment, comprenant les étapes suivantes :
a) on dispose d'au moins un moyen de diffusion capillaire pourvu d'au moins une zone de dépôt ou réception, et d'une zone de détection, au moins un réactif de capture étant immobilisé dans la zone de détection,
b) on dépose, séparément dans la zone de dépôt du moyen de diffusion capillaire-au moins une fois l'échantillon liquide,
c) on attend un temps suffisant pour la migration par diffusion capillaire de l'échantillon liquide jusqu'à la zone comportant le premier réactif de liaison spécifique, conjugué à un marqueur visible et/ou mesurable,
d) on attend un temps suffisant pour la migration par diffusion capillaire du premier réactif de liaison spécifique conjugué à un marqueur visible et/ou mesurable et de l'échantillon liquide jusqu'à la zone de détection,
e) on observe la mesure dans laquelle le réactif de liaison spécifique conjugué à un marqueur visible et/ou mesurable se fixe dans la zone de détection, pour la détermination de l'analyte simultanément par un test sandwich et par un test de compétition.

Selon un mode de réalisation, le premier réactif de liaison spécifique conjugué à un marqueur visible et/ou mesurable est déposé, à l'état sec et libre, dans ou sur le support solide poreux.

Selon un mode de réalisation, le premier réactif de liaison spécifique conjugué à un marqueur visible et/ou mesurable est ajouté extemporanément, sous forme de réactif sous forme liquide, simultanément, postérieurement ou préalablement au dépôt de l'analyte.

Selon un mode de réalisation, un diluant sous forme liquide est également ajouté ou déposé sur le support solide poreux.

Selon un mode de réalisation du procédé, à l'étape a), la zone de détection comprend au moins deux réactifs de capture, l'un des deux étant. l'analyte ou un analogue de l'analyte immobilisé, et l'autre des deux étant un réactif de liaison spécifique de l'analyte immobilisé.

Par « réactif de capture», on entend toute entité chimique biochimique ou biologique susceptible de se lier spécifiquement avec l'analyte,
ou un analogue de l'analyte.

Dans le cas d'un test par compétition, le réactif de capture se lie également au premier réactif de liaison. L'analyte et le réactif de capture forment typiquement un couple ligand/anti-ligand, antigène/anticorps, ADN/ARN ou ADN/ADN. Ainsi, si l'analyte est un antigène ou un haptène, le réactif de capture est par exemple un anticorps spécifique de l'analyte. Si l'analyte est un anticorps, le réactif de capture est l'antigène reconnu par l'anticorps ou un anticorps reconnaissant spécifiquement l'analyte. Si l'analyte est un acide nucléique, le réactif de capture est par exemple une sonde ADN complémentaire.

Le réactif de capture immobilisé est par exemple un anticorps polyclonal ou monoclonal ayant une forte affinité pour l'analyte, et plus particulièrement il peut s'agir d'un anticorps monoclonal.

Pour augmenter la sensibilité, on peut avoir recours par exemple, à un anticorps marqué selon des techniques connues de l'homme de l'art pour une détection indirecte, comme par exemple un anticorps biotinylé, permettant indirectement une détection par la formation des entités avidine-biotine et streptavidine-biotine.

Cet anticorps marqué et biotinylé peut également, soit être directement déjà déposé sur une ligne-test, dans la zone de détection, pour augmenter la sensibilité, soit être déposé avec le premier anticorps spécifique et être élué et fixé de manière que l'analyte et l'anticorps, pour augmenter le temps de contact et encore la sensibilité notamment, par exemple, en raison du nombre de sites de fixation.

Le réactif de capture spécifique de l'analyte est immobilisé sur le support solide selon des techniques connues de l'homme du métier. Ce réactif de capture est immobilisé de telle façon qu'il ne soit pas mobile à l'état humide. Cette immobilisation peut s'effectuer par exemple par absorption ou par un couplage covalent.

Par « anti-analyte », on entend toute entité chimique, biochimique, ou biologique, susceptible de se lier spécifiquement avec l'analyte ,ou avec le réactif de capture, en compétition avec l'analyte, par exemple un anticorps, un antigène ou un acide nucléique.

Selon un mode de réalisation, l'invention concerne également un procédé de détermination d'un analyte dans un échantillon liquide, comprenant les étapes suivantes :
a) on dispose d'un dispositif tel que défini précédemment,
b) on dépose un échantillon liquide dans la zone de dépôt ou réception (3) du moyen de diffusion capillaire (1) du dispositif de l'étape (a) ;
c) on attend un temps suffisant pour la migration par diffusion capillaire de l'échantillon liquide depuis la zone de dépôt ou réception (3) jusqu'à la zone de détection (5), entraînant le premier anticorps spécifique de l'analyte, conjugué à un marqueur visible et/ou mesurable (4) ;
d) on observe la mesure dans laquelle le premier anticorps spécifique de l'analyte conjugué à un marqueur visible et/ou mesurable (4) se fixe dans la zone de détection (5), pour la détermination de l'analyte, simultanément par un test sandwich et par un test de compétition. Dans un mode de réalisation avantageux, à l'étape b) on dépose, un échantillon liquide et un diluant dans la zone de dépôt ou réception (3) du moyen de diffusion capillaire (1) du dispositif de l'étape (a).

Par « analyte », on entend toute entité chimique, biochimique, ou biologique, que l'on souhaite détecter dans un échantillon. Parmi les analytes détectés par les dispositifs et les procédés selon la présente invention, on citera notamment les protéines, les peptides, les anticorps, les hormones, les stéroïdes, les antigènes dérivés d'agents infectieux ou de cellules tumorales, les agents infectieux tels que les bactéries, les virus ou les parasites, les acides nucléiques (ADN ou ARN), les composés thérapeutiques, les drogues ou encore les antibiotiques.

Par « détecter » ou « déterminer », on entend la détermination de la présence ou de l'absence d'un analyte dans un échantillon, mais aussi la mesure et la quantification d'un analyte dans un échantillon. En effet, les performances des dispositifs et procédés selon l'invention autorisent la réalisation de mesures quantitatives ou semi quantitatives.

Dans un mode de réalisation particulier de l'invention, l'analyte est la hCG (hormone choriogonadotropine), la protéine C-réactive ou l'albumine.

Par « échantillon liquide », on entend tout échantillon dans lequel l'analyte recherché est en solution ou en suspension. Cet échantillon liquide peut notamment être tout fluide biologique ou corporel. L'échantillon liquide peut également avoir été obtenu directement ou indirectement à partir d'un fluide biologique ou corporel. L'échantillon peut également être un extrait liquide d'un échantillon solide.

Typiquement, l'échantillon liquide est de l'urine, du sang total, du plasma ou du sérum.

Dans certains procédés selon la présente invention, un diluant est utilisé lorsque l'échantillon liquide est du plasma, du sérum ou du sang total par exemple. Le diluant est déposé sur le support solide poreux avec l'échantillon. Alternativement, le diluant est déposé sur le support solide poreux avant ou après l'échantillon. Ce diluant migre dans le support solide, entraînant, ou facilitant la migration de l'échantillon dans le support poreux, avec le réactif de liaison marqué. Typiquement ce diluant est composé d'une solution saline tamponnée, il peut également comprendre un détergent ou tout autre composant nécessaire à la réaction.

Par « moyen de diffusion capillaire », on entend un support solide poreux permettant la migration d'un liquide par simple diffusion capillaire. La porosité de ce support permet la diffusion capillaire (ou migration latérale) de l'échantillon et/ou des réactifs à l'état liquide ou humide. De tels moyens de diffusion capillaire sont très largement utilisés dans toutes les techniques d'immunochromatographie à migration latérale notamment.

Ainsi, les moyens de diffusion capillaire mis en oeuvre dans les dispositifs immunochromatographiques selon l'invention sont bien connus de l'homme du métier (cf. EP 0 284 232). A titre d'exemple, ces moyens de diffusion capillaire peuvent être constitués de divers supports immunochromatographiques, par exemple de cellulose, de nylon, de nitrocellulose, de polyéthylène ou de fibre de verre.

Le moyen de diffusion capillaire peut être constitué d'une ou de plusieurs parties distinctes. Les différentes parties du support pouvent être constituées de matériaux différents. Lorsque le moyen de diffusion capillaire est constitué de différentes parties ou de différents matériaux, ces éléments sont disposés de telle façon à permettre la continuité de l'écoulement capillaire dans le moyen de diffusion capillaire.

Typiquement, le moyen de diffusion capillaire est constitué d'un support solide poreux allongé selon la direction de diffusion capillaire. De préférence, le moyen de diffusion capillaire des dispositifs selon l'invention comprend un support solide poreux en forme de bandelette immunochromatographique. Le moyen de diffusion capillaire se présente par exemple sous la forme d'une bandelette immunochromatographique constituée de plusieurs bandelettes superposées ou chevauchantes.

Le dispositif selon l'invention peut par exemple être constitué d'une bandelette chromatographique fixée sur un support rigide. Le support rigide peut être constitué de matériaux divers tel que du carton, du carton plastifié ou plus préférentiellement de matières plastiques. De préférence, le support rigide est constitué de polystyrène.

Le moyen de diffusion capillaire comprend une zone de dépôt ou de réception de l'échantillon, et une zone de détection de l'échantillon. La zone de dépôt et la zone de détection sont des zones distinctes et séparées du moyen de diffusion capillaire. Ces zones sont disposées de façon à permettre la continuité de l'écoulement capillaire depuis la zone de dépôt ou réception, jusqu'à la zone de détection selon une direction de diffusion capillaire. Typiquement, la zone de dépôt et la zone de détection correspondent respectivement à l'une et l'autre des extrémités opposées du moyen de diffusion capillaire. Le moyen de diffusion capillaire est ainsi par exemple constitué d'un support solide poreux allongé selon la direction de diffusion capillaire, présentant une extrémité proximale et une extrémité distale constituant respectivement la zone de dépôt et la zone de détection.

Ces zones peuvent par exemple être présentes sur une même bande constituée d'un matériau unique. Avantageusement, un matériau poreux spécifique correspond à chaque zone du moyen de diffusion capillaire. Un matériau absorbant poreux peut par exemple être utilisé pour la zone de dépôt de l'échantillon. En effet, la zone de dépôt du moyen de diffusion capillaire est destinée à être mise en contact avec un flux d'urine ou à recevoir un échantillon liquide. On choisira donc un matériau absorbant adapté. Ces matériaux sont bien connus de l'homme du métier. Et un autre matériau absorbant peut être mis en oeuvre pour la zone recevant le premier réactif de liaison spécifique, et/ou la zone de détection.

La zone de dépôt de l'échantillon du moyen de diffusion capillaire peut coopérer avec un organe de captation en matériau absorbant. Cet organe de captation peut être directement mis en contact avec un flux d'urine par exemple. Comme décrit dans WO 00/00288, l'organe de captation peut être mobile entre deux positions, l'une de recueil de l'échantillon liquide, à l'écart du moyen de diffusion capillaire, et l'autre en continuité ou contact capillaire avec la zone de dépôt du moyen de diffusion capillaire.

Le moyen de diffusion capillaire porte par exemple un premier anticorps spécifique de l'analyte conjugué à un marqueur visible et/ou mesurable. Cet anticorps est déposé à l'état sec dans le moyen de diffusion capillaire, et est libre de migrer par diffusion capillaire à l'état humide. Ce premier anticorps spécifique de l'analyte peut être localisé dans la zone de dépôt ou réception de l'échantillon. Mais, ce premier anticorps peut aussi être déposé à l'état sec, en aval de la zone de dépôt ou réception de l'échantillon, pour toute perte de réactif par un effet de lavage lors du dépôt de l'échantillon. Ainsi, l'échantillon liquide est déposé dans la zone de dépôt du moyen de diffusion capillaire, puis cet échantillon migre par diffusion capillaire à travers le moyen de diffusion capillaire, entraînant alors le premier anticorps spécifique de l'analyte conjugué à un marqueur.

Avantageusement, le moyen de diffusion capillaire peut être incorporé dans un support de préhension. Ce support de préhension facilite la manipulation du moyen de diffusion capillaire, et peut également protéger celui-ci notamment de l'humidité.

Le support de préhension peut envelopper partiellement ou totalement le moyen de diffusion capillaire.

Le support de préhension peut être constitué de matériaux divers tel que du carton, du carton plastifié ou plus préférentiellement de matières plastiques. De façon avantageuse, le support de préhension est constitué d'un matériau rigide et imperméable. Ces supports de préhension ou boîtiers sont notamment décrits dans les brevets EP 0 291 194, EP 0560411, EP 0 560 410 et EP 1 091 808.

Habituellement, le support de préhension est en forme de boîtier.

Dans un mode de réalisation de l'invention, le moyen de diffusion capillaire peut comprendre une zone de dépôt ou recueil de l'échantillon, saillante par rapport au support de préhension, pour la réception de l'échantillon liquide.

Dans un autre mode de réalisation de l'invention, le support de préhension ou le boîtier comprend au moins une ouverture pour le dépôt de l'échantillon liquide.

Typiquement, le support de préhension est également pourvu d'au moins une fenêtre d'observation pour observer la zone de détection du moyen de diffusion capillaire.

Les réactifs mis en oeuvre dans les procédés selon la présente invention, permettant la détermination de l'analyte dans l'échantillon liquide, sont bien connus de l'homme du métier.

Le premier anticorps spécifique de l'analyte, conjugué à un marqueur visible et/ou mesurable, est déposé à l'état sec dans ou sur le moyen de diffusion capillaire, mais il est libre de migrer par diffusion capillaire à l'état humide.

Le deuxième anticorps spécifique de l'analyte est immobilisé de façon permanente dans la zone de détection du moyen de diffusion capillaire.

Par « anticorps spécifique de l'analyte », on entend un anticorps capable de se lier spécifiquement avec l'analyte dans une liaison de type antigène/anticorps. Il s'agit typiquement d'un anticorps polyclonal ou monoclonal ayant une forte affinité pour l'analyte. De préférence, il s'agit d'un anticorps monoclonal.

Le deuxième anticorps spécifique de l'analyte est immobilisé dans la zone de détection du moyen de diffusion capillaire selon des techniques connues de l'homme du métier. Ce deuxième anticorps est immobilisé de telle façon qu'il ne soit pas mobile à l'état humide. Cette immobilisation peut s'effectuer par exemple par absorption ou par un couplage covalent.

Le premier et le deuxième anticorps se lient respectivement et spécifiquement avec l'analyte, par exemple sur deux sites épitopiques, identiques ou différents de l'analyte.

Le premier et le deuxième anticorps des dispositifs de la présente invention permettent la détermination de l'analyte dans l'échantillon liquide par un test sandwich.

En outre, les dispositifs selon la présente invention comprennent l'analyte lui-même, ou un analogue de l'analyte, immobilisé dans la zone de détection du moyen de diffusion capillaire.

L'analyte, ou l'analogue de l'analyte, est immobilisé dans la zone de détection du moyen de diffusion capillaire selon des techniques connues de l'homme du métier. L'analyte, ou l'analogue de l'analyte, est immobilisé de telle façon qu'il ne soit pas mobile à l'état humide. Cette immobilisation peut s'effectuer par exemple par absorption ou par un couplage covalent.

Ce second réactif de la zone de détection est identique à l'analyte lui-même ou un analogue approprié de l'analyte. Par analogue approprié de l'analyte, on entend toute entité se liant de manière spécifique au premier réactif de liaison ou anticorps spécifique de l'analyte, en compétition avec l'analyte.

Dans un mode de réalisation, d'une part, le premier anticorps spécifique de l'analyte, conjugué à un marqueur visible et/ou mesurable, et d'autre part l'analyte, ou l'analogue de l'analyte, immobilisé dans la zone de détection du moyen de diffusion capillaire, permettent la détection de l'analyte de l'échantillon liquide par un test de compétition. Le dépôt du deuxième anticorps spécifique de l'analyte est effectué en amont de l'analyte, ou de l'analogue de l'analyte, par rapport à la direction de diffusion capillaire.

Le premier anticorps spécifique de l'analyte, libre de migrer à l'état humide, est conjugué à un marqueur visible et/ou mesurable, permettant une mesure ou une observation directe du résultat, et du test sandwich est du test de compétition. Tout marqueur visible peut être observé directement à l'oeil nu lorsqu'il est concentré dans la zone de détection du moyen de diffusion capillaire. La mesure du marqueur peut s'effectuer directement à l'oeil nu, ou à l'aide d'un appareil de mesure. Cette mesure se fait par une observation directe ne nécessitant pas de manipulation supplémentaire.

Avantageusement, les dispositifs selon la présente invention comprennent un premier anticorps spécifique de l'analyte conjugué à un marqueur visible et/ou mesurable.

Les marqueurs retenus selon la présente invention permettent une détection directe à l'oeil nu, ou indirecte à l'aide d'un appareil, en raison de l'émission d'un signal, ledit signal étant par exemple, une fluorescence, une coloration, une présence d'isotope, un signal magnétique.

On citera par exemple les marqueurs particulaires, colorés ou fluorescents, constitués de particules de petite taille insolubles dans l'eau et qui forment donc des suspensions, dispersions ou sols, en phase liquide.

Les marqueurs particulaires sont bien connus de l'homme du métier. On connaît notamment les marqueurs particulaires colorés ou fluorescents. A titre d'exemple, on citera l'or colloïdal, les particules de latex colorées, les particules de latex fluorescentes et les particules conjuguées à l'avidine ou à la streptavidine.

Parmi les marqueurs permettant une observation directe à l'oeil nu, on citera aussi les marqueurs de type dextran (Hansen T.M., IVD Technology 4, 35-40, 2003). Le réactif de liaison est alors conjugué à une chaîne de dextran (dérivé de polysaccharide) portant des fluorophores.

Les réactifs de liaison sont conjugués au marqueur visible et/ou mesurable selon des techniques bien connues de l'homme du métier.

Dans un mode de réalisation préféré de l'invention, la zone de détection du moyen de diffusion capillaire comprend un troisième réactif disposé en aval du deuxième anticorps spécifique de l'analyte, et en aval de l'analyte, ou de l'analogue de l'analyte, par rapport à la direction de diffusion capillaire. Ce troisième réactif permet de disposer d'un contrôle positif afin de s'assurer de la diffusion effective capillaire de l'échantillon liquide depuis la zone de dépôt jusqu'à la zone de détection du moyen de diffusion capillaire.

Ce troisième réactif est immobilisé de façon permanente dans la zone de détection du moyen de diffusion capillaire.

Il peut s'agir par exemple d'un anticorps se liant au premier anticorps spécifique de l'analyte conjugué au marqueur. Dans ce cas, ce troisième réactif permet de vérifier la migration du premier anticorps spécifique de l'analyte conjugué au marqueur, jusqu'à la zone de détection du moyen de diffusion capillaire. Alternativement, ce troisième réactif est indépendant de l'analyte et permet simplement de vérifier la diffusion de l'échantillon liquide jusqu'à la zone de détection.

L'invention est décrite plus en détail par référence aux figures suivantes :

### Figures

Figure 1 : Courbe signal/concentration de l'analyte pour un dosage immunologique par un test sandwich
Figure 2 : Courbe signal/concentration de l'analyte pour un dosage immunologique par un test de compétition
Figure 3 : Dispositif de dosage simultané d'un analyte par un test selon la présente invention
Figure 4 : Dispositif de test rapide selon la présente invention, comprenant une bandelette immunochromatographique dans un boîtier.
Figure 5 : Bandelette immunochromatographique pour la détection de l'hCG
Figure 6 : Résultat du test selon Figure 5 en cas de concentration faible en analyte
Figure 7 : Résultat du test selon Figure 5 en cas de concentration forte en analyte

La figure 1 représente la courbe signal/concentration obtenue pour un dosage immunologique par test sandwich. La courbe obtenue est une courbe du type courbe de Gauss, qui montre qu'à un signal donné (S) correspondent deux concentrations possibles de l'analyte (C1 et C2), l'une faible (C1) et l'autre élevée (C2) lors de la lecture du résultat à un temps défini.

La figure 2 représente une courbe signal/concentration pour un dosage par compétition. On observe l'obtention de deux signaux différents (S1 et S2) pour deux concentrations (C1 et C2) de l'analyte à doser. Cependant, on observe une extinction du signal à des concentrations relativement peu élevées d'analyte.

La figure 3 représente un dispositif de dosage simultané d'un analyte selon la présente invention, la vue A) présentant le dispositif sans support de préhension et la vue B), le dispositif comprenant un support de préhension.

La vue 3A) représente le dispositif comportant un moyen de diffusion capillaire (1) comprenant dans la direction de diffusion capillaire (2) :
a) une zone de dépôt ou réception de l'échantillon (3) sur un premier matériau capillaire 20 ;
b) un premier anticorps spécifique de l'analyte (4), conjugué à un marqueur visible et/ou mesurable, libre de migrer par diffusion capillaire à l'état humide dans le moyen de diffusion capillaire (1) ;ce premier anticorps marqué est déposé, à l'état sec et libre, sur un deuxième matériau capillaire 21, en continuité d'écoulement capillaire avec le premier matériau capillaire 20 ;
c) une zone de détection de l'analyte (5) comprenant successivement, dans la direction de diffusion capillaire (2), d'une part un deuxième anticorps (6) spécifique de l'analyte immobilisé, et d'autre part l'analyte, ou un analogue de l'analyte, immobilisé (7) ; le deuxième anticorps et l'analyte (ou l'analogue de l'analyte) sont disposés ou déposés sur un troisième matériau capillaire, en continuité d'écoulement capillaire avec le deuxième matériau capillaire 21 ;

La vue B) représente le moyen de diffusion capillaire (1) intégré dans un support de préhension (8) pourvu d'au moins une fenêtre d'observation (9) permettant d'observer la zone de détection (5), et d'une ouverture 23 de recueil, dépôt, ou réception de l'échantillon liquide.

### Exemples

Test de grossesse (détection dans l'urine de l'hormone choriogonadotropine ou hCG)

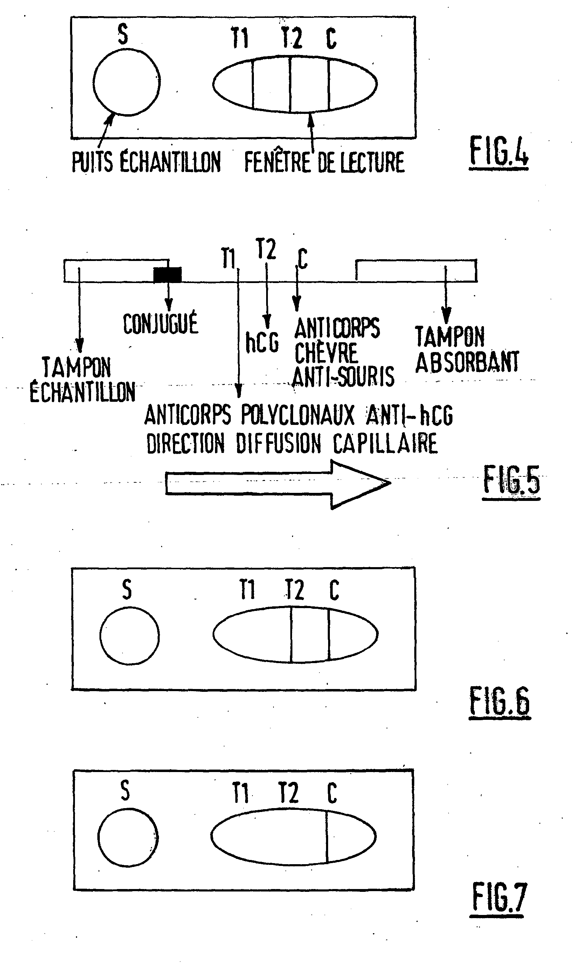

La figure 4 représente la vue extérieure d'un test rapide de type boîtier dans lequel est insérée une bandelette chromatographique selon la présente invention.

La bandelette 1 contient un anticorps monoclonal anti β-hCG 4 marqué à l'or colloïdal, déposé sur de la fibre de verre 21 et déshydraté (conjugué). Ce marqueur particulaire est dissout par l'échantillon liquide lors de son dépôt dans le puits échantillons (S), puis entre en contact successivement, par migration sur la bandellette, avec une première zone contenant un anticorps polyclonal anti-hCG fixé (T1), une deuxième zone contenant de l'hCG fixée (T2), et une troisième zone contenant un anticorps polyclonal de chèvre anti Immunoglobuline de souris (C), voir Figure 5.

En cas d'absence (ou concentration très faible) d'hormone hCG dans l'échantillon déposé, pratiquement aucune réaction visible ne se produira dans la zone T1, alors qu'une ligne apparaîtra dans la zone T2 et la zone C (Figure 6). En cas de très forte concentration en hCG dans l'échantillon, la réaction dans la zone T1 sera très faible ou inexistante (à cause de l'effet "crochet" ou "hook" précédemment décrit) ; aucune réaction visible ne se produira dans la zone T2, car la quasi-totalité de l'anticorps anti β-hCG marqué à l'or colloïdal ayant réagi avec l'analyte (hCG) de l'échantillon, n'est plus disponible pour réagir avec l'analyte (ou tout analogue de l'analyte), et la zone C sera positive (Figure 7).

Ce système permet donc effectivement de différencier l'interprétation du résultat en cas de faible concentration d'analyte (2 lignes positives : T2+C) ou de forte concentration d'analyte (1 ligne positive : C). De plus l'apparition des lignes se fera à des endroits différents (T1 ou T2).

Le tableau ci-dessous résume les résultats obtenus dans la zone de détection (T1, T2 et C) en fonction de la concentration en analyte dans l'échantillon à l'aide d'une lecture visuelle du test.

**Tableau 1 : Résultats de la lecture visuelle du test en fonction de la concentration en hCG dans l'échantillon.**

| Concentration en hCG (mIU/ml) | | 0 | 5 | 50 | 00 | .10³ | .10⁴ | 0⁵ | .10⁵ |
|---|---|---|---|---|---|---|---|---|---|
| Ligne T1 | | /- | | + | + | + | + | + | |
| Ligne T2 | + | + | + | + | + | + | + | | |
| Ligne C | + | + | + | + | + | + | + | + | + |

En plus d'une interprétation visuelle des résultats, il est tout à fait possible de réaliser une lecture à l'aide d'un automate, qui est alors capable de donner un résultat quantitatif ou semi quantitatif de la concentration de l'analyte, sans dilution préalable de l'échantillon et sans faire de gammes de concentration. En effet en fonction de la zone de concentration, les résultats obtenus dans la zone de détection sont différents et l'interprétation de cette différence permet après étalonnage ou par observation visuelle de quantifier les concentrations de l'analyte.

## Revendications

1. Dispositif de détermination d'un analyte dans un échantillon liquide, comportant :
- un moyen de diffusion capillaire (1), par migration latérale déterminant une direction de référence (2) de diffusion capillaire, comportant une zone de dépôt (3) de l'échantillon liquide, et une zone de détection (5) de l'analyte, disposée en aval de ladite zone de dépôt (3),
- un premier réactif (4) de liaison spécifique de l'analyte, conjugué à un marqueur visible et/ou mesurable, libre de migrer à l'état humide, par diffusion capillaire, dans le moyen de diffusion capillaire (1), selon la direction de référence, la zone de disposition du premier réactif de liaison étant confondue avec la zone de dépôt (3) de l'échantillon, ou étant disposée en aval de cette dernière, mais en amont de la zone de détection (5),
- un deuxième réactif (6) de liaison spécifique de l'analyte, immobilisé dans la zone de détection (5),
**caractérisé en ce que** la zone de détection (5) comporte un troisième réactif constitué par l'analyte (7), ou un analogue de l'analyte, immobilisé, et disposé à distance du deuxième réactif (6) de liaison spécifique, en aval de celui-ci.

2. Dispositif selon la revendication 1, **caractérisé en ce qu'**il comporte un organe de captation de l'échantillon liquide, indépendant du moyen de diffusion capillaire, susceptible d'être amené en contact de la zone de dépôt, pour un écoulement dudit échantillon dans ladite zone de dépôt.

3. Dispositif selon la revendication 1, **caractérisé en ce** le moyen de diffusion capillaire (1) comporte une zone de disposition du premier réactif (4) de liaison, à l'état sec et libre, dans ou sur ledit moyen de diffusion capillaire.

4. Dispositif selon la revendication 1, **caractérisé en ce que** le deuxième réactif (6) de liaison spécifique, et/ou l'analyte (7), ou l'analogue de l'analyte, sont disposés sur le moyen de diffusion capillaire selon une ligne transversale à la direction de référence.

5. Dispositif selon la revendication 1, **caractérisé en ce que** le moyen de diffusion capillaire comporte une zone de contrôle (c), en aval de la zone de détection (5).

6. Procédé de détermination d'un analyte dans un échantillon liquide par mise en oeuvre d'un dispositif selon l'une quelconque des revendications 1 à 5, **caractérisé en ce que** :
- on dépose de l'échantillon liquide dans la zone de dépôt (3) du moyen de diffusion capillaire (1),
- on attend un temps suffisant pour la migration par diffusion capillaire de l'échantillon liquide jusqu' à la zone de détection (5),
- on observe dans la zone de détection (5) la mesure dans laquelle, d'une part le premier réactif (4) de liaison spécifique complexé avec l'analyte se fixe au deuxième réactif (6) de liaison spécifique, en obtenant un premier signal, et d'autre part le premier réactif (4) de liaison non complexé se fixe au troisième réactif constitué par l'analyte (7) ou un analogue de l'analyte, en obtenant un deuxième signal,
- on détermine l'analyte à partir des premier et deuxième signaux.

7. Procédé selon la revendication 6, **caractérisé en ce que** la quantité du premier réactif (4) de liaison est excédentaire par rapport à la quantité de l'analyte présente dans l'échantillon liquide, ou du nombre de sites de complexation présents dans ledit échantillon liquide.

8. Procédé selon la revendication 6, **caractérisé en ce que** une concentration faible en analyte est détectée par l'absence du premier signal et la présence du second signal.

9. Procédé selon la revendication 6, **caractérisé en ce que** une concentration importante en analyte est détectée par l'absence des deux signaux.

## Claims

1. Device for determining an analyte in a liquid sample, comprising:
- a means for capillary diffusion (1), by lateral migration determining a capillary diffusion reference direction (2), comprising a depositing zone (3) for depositing the liquid sample, and a detecting zone (5) for detecting the analyte, placed downstream of said depositing zone (3),
- a first reagent (4) for specific binding of the analyte, conjugated to a visible and/or measurable label, free to migrate in the moist state, by capillary diffusion, in the means for capillary diffusion (1), along the reference direction, the zone for placing the first binding reagent being combined with the depositing zone (3) for depositing the sample, or being placed downstream of the latter zone, but upstream of the detecting zone (5),
- a second reagent (6) for specific binding of the analyte, immobilized in the detecting zone (5),
**characterized in that** the detecting zone (5) comprises a third reagent constituted of the analyte (7), or an analogue of the analyte, which is immobilized and is placed at a distance from the second reagent (6) for specific binding, downstream of the latter.

2. Device according to Claim 1, **characterized in that** it comprises a member for taking up the liquid sample, independent of the means for capillary diffusion, capable of being brought into contact with the depositing zone, in order for said sample to flow into said depositing zone.

3. Device according to Claim 1, **characterized in that** the means for capillary diffusion (1) comprises a zone for placing the first binding reagent (4), in the dry and free state, in or on said means for capillary diffusion.

4. Device according to Claim 1, **characterized in that** the second reagent (6) for specific binding, and/or the analyte (7), or the analogue of the analyte, are placed on the means for capillary diffusion along a line transverse to the reference direction.

5. Device according to Claim 1, **characterized in that** the means for capillary diffusion comprises a controlling zone (c) downstream of the detecting zone (5).

6. Method for determining an analyte in a liquid sample by using a device according to any one of Claims 1 to 5, **characterized in that**:
- the liquid sample is deposited in the depositing zone (3) of the means for capillary diffusion (1),
- a period of time sufficient for the migration by capillary diffusion of the liquid sample to the detecting zone (5) is allowed to elapse,
- in the detecting zone (5), the measurement in which, firstly, the first reagent (4) for specific binding, complexed with the analyte, binds to the second reagent (6) for specific binding, with a first signal being obtained, and, secondly, the noncomplexed first binding reagent (4) binds to the third reagent constituted of the analyte (7) or an analogue of the analyte, with a second signal being obtained, is observed,
- the analyte is determined from the first and second signals.

7. Method according to Claim 6, **characterized in that** the amount of the first binding reagent (4) is in excess compared with the amount of the analyte present in the liquid sample, or the number of complexation sites present in said liquid sample.

8. Method according to Claim 6, **characterized in that** a low concentration of analyte is detected through the absence of the first signal and the presence of the second signal.

9. Method according to Claim 6, **characterized in that** a high concentration of analyte is detected through the absence of the two signals.

## Patentansprüche

1. Vorrichtung zum Bestimmen eines Analyten in einer flüssigen Probe, mit:
- einem Mittel (1) zur Kapillardiffusion durch seitliche Migration, die eine Referenzrichtung (2) der Kapillardiffusion bestimmt, das eine Zone (3) für die Ablagerung der flüssigen Probe und eine Zone (5) für die Detektion des Analyten, die stromabseitig der Ablagerungszone (3) angeordnet ist, aufweist,
- einem ersten Bindungsreagenz (4), das für den Analyten spezifisch ist und mit einem sichtbaren und/oder messbaren Markierer gekoppelt ist, der im feuchten Zustand durch Kapillardiffusion längs der Referenzrichtung frei in das Kapillardiffusionsmittel (1) migrieren kann, wobei die Ablagerungszone des ersten Bindungsreagenz mit der Ablagerungszone (3) der Probe zusammenfällt oder stromabseitig dieser Letzteren, jedoch stromaufseitig der Detektionszone (5) angeordnet ist,
- einem zweiten Bindungsreagenz (6), das für den Analyten spezifisch und das in der Detektionszone (5) unbeweglich gemacht ist,
**dadurch gekennzeichnet, dass** die Detektionszone (5) ein drittes Reagenz aufweist, das durch den Analyten (7) oder durch einen zu dem Analyten analogen Stoff gebildet ist, das bzw. der unbeweglich gemacht und in einem Abstand von dem zweiten spezifische Bindungsreagenz (6) stromabseitig dieses Letzteren angeordnet ist.

2. Vorrichtung nach Anspruch 1, **dadurch gekennzeichnet, dass** sie ein Organ zum Einfangen der flüssigen Probe unabhängig von dem Kapillardiffusionsmittel aufweist, das in Kontakt mit der Ablagerungszone gebracht werden kann, damit die Probe in die Ablagerungszone fließen kann.

3. Vorrichtung nach Anspruch 1, **dadurch gekennzeichnet, dass** das Kapillardiffusionsmittel (1) eine Zone (4) für die Ablagerung des ersten Bindungsreagenz im trockenen und freien Zustand in oder auf dem Kapillardiffusionsmittel aufweist.

4. Vorrichtung nach Anspruch 1, **dadurch gekennzeichnet, dass** das zweite spezifische Bindungsreagenz (6) und/oder der Analyt (7) oder ein zu dem Analyten analoger Stoff auf dem Kapillardiffusionsmittel auf einer zu der Referenzrichtung transversalen Linie angeordnet sind.

5. Vorrichtung nach Anspruch 1, **dadurch gekennzeichnet, dass** das Kapillardiffusionsmittel eine Kontrollzone (c) stromabseitig der Detektionszone (5) aufweist.

6. Verfahren zum Bestimmen eines Analyten in einer flüssigen Probe zum Betreiben einer Vorrichtung nach einem der Ansprüche 1 bis 5, **dadurch gekennzeichnet, dass**:
- die flüssige Probe in der Ablagerungszone (3) des Kapillardiffusionsmittels (1) abgelagert wird,
- eine Zeit gewartet wird, die für die Migration durch Kapillardiffusion der flüssigen Probe in die Detektionszone (5) ausreicht,
- in der Detektionszone (5) die Messung beobachtet wird, in der einerseits das erste spezifische Bindungsreagenz (4), das zusammen mit dem Analyten zu einem Komplex gemacht worden ist, sich an dem zweiten spezifischen Bindungsreagenz (6) fixiert, wobei ein erstes Signal erhalten wird, und andererseits das erste Bindungsreagenz (4), das nicht zu einem Komplex gemacht worden ist, sich an dem dritten Reagenz fixiert, das durch den Analyten (7) oder durch einen zu dem Analyten analogen Stoff gebildet ist, wobei ein zweites Signal erhalten wird,
- der Analyt anhand des ersten und des zweiten Signals bestimmt wird.

7. Verfahren nach Anspruch 6, **dadurch gekennzeichnet, dass** die Menge des ersten Bindungsreagenz (4) höher ist als die Menge des in der flüssigen Probe vorhandenen Analyten oder als die Anzahl von Komplexbildungsstellen, die in der flüssigen Probe vorhanden sind.

8. Verfahren nach Anspruch 6, **dadurch gekennzeichnet, dass** bei Abwesenheit des ersten Signals und bei Vorhandensein des zweiten Signals eine geringe Konzentration des Analyten detektiert wird.

9. Verfahren nach Anspruch 6, **dadurch gekennzeichnet, dass** bei Abwesenheit der zweiten Signale eine große Konzentration des Analyten detektiert wird.
